# EUROPEAN PATENT APPLICATION

(11) **EP 0 880 936 A2**
(43) Date of publication of application: **02.12.1998**
(21) Application number: 97118979.0
(22) Date of filing: 30.10.1997
(51) Int. Cl.: A61B 5/00

(54) **Monitoring physical condition of a patient by telemetry**

(30) Priority: 29.05.1997 JP 139805/97; 06.10.1997 JP 272651/97
(71) Applicant: Akai, Koji, Kamakura-shi, Kanagawa-ken (JP)
(72) Inventor: Akai, Koji, Kamakura-shi, Kanagawa-ken (JP)
(74) Representative: Wagner, Karl H., Dipl.-Ing.

(57) **Abstract**

A system is provided for remotely monitoring the current location and physical condition/parameters of a specific person on a real time basis. The monitored physical parameters include the blood glucose level. In accordance with the invention, the detection of a blood glucose level can be effected in an un-invasive manner, which is quite different from the prior art method which would be painful. A terminal unit attached onto the person's body is, via a radio network, in communication with a central unit located at a medical institute of the area. The detection of vital signs including a blood glucose level is continuously and intermittently effected by the terminal unit. Once an emergency situation arises, an alarm signal will automatically be transmitted to the central unit regardless of the action and intention of the person.

## Description

The present invention relates to a monitoring system by which the physical condition of a human being including his/her blood glucose level and the physical location of the human being may be monitored. A person to be monitored by the monitoring system of the invention includes those who need to be continuously monitored, those who may suffer from a sudden physical disorder and those who may have poriomania.

As the ratio of elderly people in the population increases, more and more people need to be monitored with respect to their physical condition and their location on a real time basis. However, a monitoring system that can be used to continuously monitor the physical condition and location of a person with a minimum amount of hindrance to him/her does not concurrently exist or is not widely known.

Due to the higher ratio of elderly people in the population and changes in life style, more people need to have their blood glucose value controlled . In measuring blood glucose value by means of a prior art apparatus, a small amount of blood is physically drawn from a vein or a finger by inserting a needle thereinto and a measuring device is directly applied to the blood. It is, however, a painful procedure and thus not easy for most people to conduct on their own finger. Further, since a blood glucose value significantly varies depending upon how many hours have passed since the last meal as well as other factors, it has not been easy to precisely measure a blood glucose value and to assess its effect on the person's body only by using blood drawn from a finger at a certain time of the day.

Further, it is not possible to use a sample of blood drawn from a finger to determine temporal changes in blood glucose over a certain time interval. For example, even if the blood glucose value calculated based on a sample taken from a finger is outside a normal range, it can not be known whether such a deviation occurred slowly or suddenly. In other words, it is not possible for a prior art measuring device to continuously monitor a person's blood glucose value and automatically detect an abnormality when the measured value deviates from a predetermined normal range.

As an example of prior art location search systems, one is known which utilizes the PHS (personal handy-phone system) network to locate a person via an antenna. However, since the area covered by a PHS antenna is quite large, it is difficult to locate the desired person within the area even if the antenna in communication with the person has been located. Thus such a system does not fulfill the purpose of enabling a rapid discovery and treatment system. Of course, it is vital to quickly attend to a person once that an emergency situation is recognized.

Even though appropriate monitoring is possible by using a device, if the use of such a device restricts everyday life and lowers the quality of life (QOL), then such a device will tend not to be used by a patient. In view of these points, it has to be said that a monitoring system which is capable of continuously monitoring the physical condition of a person while not interfering with the ordinary activities of the monitored person does not yet exist.

Considering the above-mentioned situation, the purpose of the present invention is to provide a blood glucose monitoring system by which it is no longer necessary to measure invasively a blood glucose level as in the prior art measuring devices and by which a shortcoming of the local sampling of the blood glucose level in the prior art measuring device is overcome. Since a blood glucose level significantly varies depending upon when the person ate, drank, etc., the prior art spot sampling at a certain point is insufficient for determining temporal changes in the level. In accordance with the monitoring system of the present invention, the blood glucose level is continuously and regularly monitored so that a rapid treatment may be given whenever a harmful deviation occurs. Further, as the blood glucose level monitoring is performed in an uninvasive manner, the psychological and physical burden resulting from monitoring can be kept to a minimum level.

Thus, in accordance with an aspect of the present invention, the blood monitoring system of the invention comprises: sensor means attached to a part of a person for detecting data representative of the blood glucose level of the person, the sensor means having a light emitter and a light receiver; data processor means for calculating the blood glucose level of the person on the basis of the light data transferred from said sensor means, the data processor means having a microprocessor incorporated for controlling said sensor means and analyzing the data received by the light receiver; and storage means for storing a database consisting of the physical data of the person, the physical data including the blood glucose level of the person for a certain time period in the past; said data processor means comprising means for comparing the calculated blood glucose level with data in the database, means for accessing, on the basis of the data comparison, possible effects that the calculated blood glucose level may have on the person's body, and means for instructing the monitoring system to generate an alarm signal when the effects exceed a predetermined threshold level that should be judged as being medically dangerous.

The alarm signal may be transmitted, via a wireless network, to medical/public health personnel at a medical institute which functions as a center for the medical network of the area in which the person lives. The person him/herself obtains blood glucose information on the display of the system. If the abnormality of the blood glucose level is known at an early stage, it is possible to take necessary measures to adjust a blood glucose level to within range.

Further, considering the state of the art situation regarding the location and physical condition monitoring system, another purpose of the present invention is to significantly reduce the time required to find a person being monitored. Also, in employing such a monitoring system, it is a purpose of the present invention to maintain a proper QOL. In addition to the monitoring of the location, an emergency alarm system is necessary. That is, while the physical condition of the person is continuously and intermittently monitored, when the parameters representative of the physical condition of the person are outside of the normal range predetermined by a physician as result of the retrieval and analysis of the past physical data of the person, then, an emergency alarm will be automatically generated regardless of the conduct and intention of the person. Such a prior art pendant-type device is not effective because people often cannot actuate the switch for this type of device because the person is in a confused state due to physical abnormality.

Past physical data must be retrieved because the normal range of a physical parameter has to be based on the specific person's individual medical history so that a more accurate and individual judgment can be made rather than that based on a general non-customized range, such as the age range of a person or based on their gender. This emergent function makes it possible for an abnormality to be detected and treated at an early stage.

In particular, the vital signs of the person being monitored are continuously or intermittently monitored for twenty-four hours a day by attaching a monitoring apparatus onto a part of the person's body. And once an abnormality is detected, an emergency signal will be automatically generated. This emergency signal will be transmitted, via a radio transmission network, to a central unit at a medical institute. It is also possible for medical personnel to locate the person and to communicate with the located person, thereby enabling better care to be taken of people being monitored at home.

The radio network between the units can be one of the existing radio communication system such as the PHS. Also, a combination of the radio paging system using portable beeper and a private network.

This monitoring system permits physicians and nurses at a medical institute to remotely monitor people at home and to react in an emergency situation. Also, this monitoring system helps a medical database to be constructed in a computer system at the medical institute, which stores physical data on the people being monitored.

In accordance with the present invention, a system for remotely monitoring the present location and physical condition of a person on a real time basis is provided and the system comprising: a central unit which is a computer system for performing remote monitoring on a real time basis; monitoring means attached to the body of the person for detecting parameters representative of the physical condition of the person; and wireless communication means located in the neighborhood of said monitoring means or integrated with said monitoring means for communicating with said central unit; wherein the monitoring unit consisting of said monitoring means and said wireless communication means is responsive to the actuation of an emergency switch by the person for transmitting or automatically transmitting, in a wireless form, a signal representative of the present location and physical condition of the person to said central unit and the monitoring unit is also responsive to the instructions from said central unit for transmitting a signal representative of the present location and physical condition of the person to said central unit and wherein said monitoring system comprises audio communication means for exchanging audio communication with said central unit.

Fig. 1 is a perspective view of a blood glucose monitoring system which is a first embodiment of the invention.

Fig. 2 shows details of the blood glucose monitoring system which is a first embodiment of the invention.

Fig. 3 is a perspective view of a location and physical condition monitoring system which is a second embodiment of the invention.

Fig. 4 shows details of the location and physical condition monitoring system which is a first embodiment of the invention.

Fig. 5 is a perspective view of a system for taking care of a person at home which is a third embodiment of the invention.

Fig. 6 shows details of the system for taking care of a person at home which is a third embodiment of the invention.

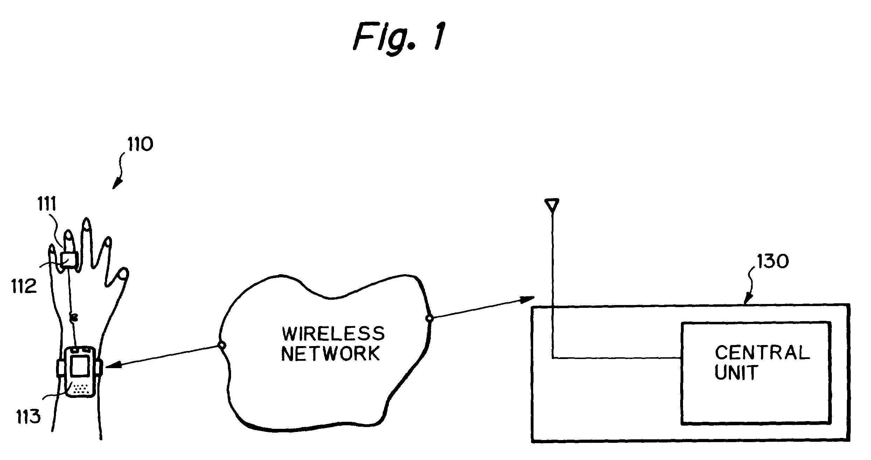

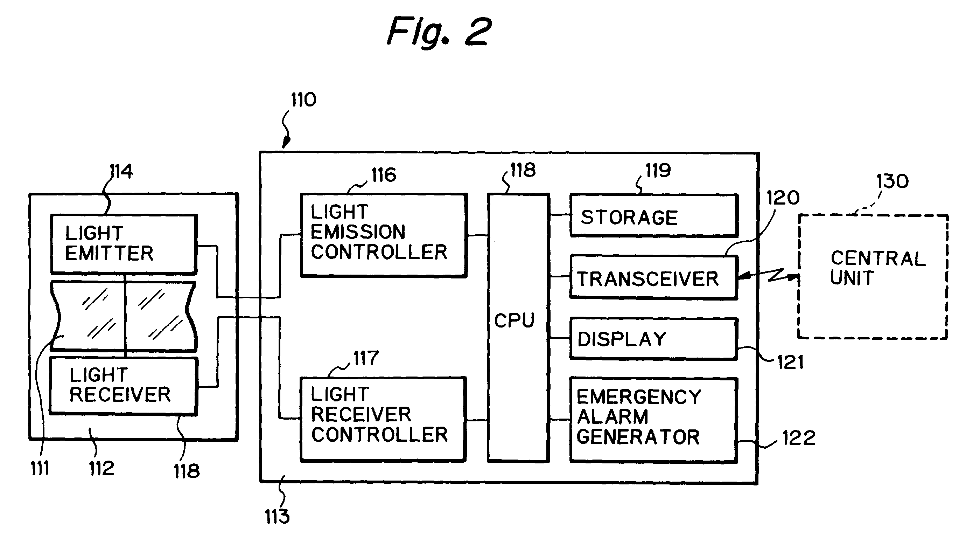

Preferred embodiments of the present invention will now be described by referring to the attached drawings. A blood glucose monitoring system which is the first embodiment of the present invention will be first described in referring to Fig. 1 and Fig. 2. Fig. 1 is a perspective illustration of blood glucose monitoring system 110 in accordance with the present invention. Blood glucose monitoring system 110 comprises sensor unit 112 attached to a person's body 111 and data processing unit 113 connected to sensor unit 112. In the embodiment shown in Fig. 1, sensor unit 112 is attached on a finger of the person. Sensor unit 112 may also be attached into an external auditory meatus. Data processing unit 113 includes a microprocessor. The data detected by sensor unit 112 is analyzed by data processing unit 113 and the blood glucose value is calculated. Then, the calculated blood glucose value is compared with the person's blood glucose data in the past and it is determined whether the detected and calculated value is within the normal range. And if necessary, an attention or alarm signal is transmitted.

Data processing unit 113 is arranged to be able to communicate with central unit 130 installed at a medical institute in an area via a wireless communication network and generates an emergency signal when necessary.

In Fig. 2, sensor unit 112 and data processing unit 113 connected to sensor unit 112, both of which belong to blood glucose monitoring system of the present invention are shown in more detail. Reference numerals that are same as in Fig. 1 are used for the same elements in Fig. 2. Sensor 112 is attached to a finger 111 of the person being monitored and consists of light emitter 114 and light receiver 115.

Data processing unit 113 includes central processing unit (CPU) 118 which is a microprocessor, controller for the light emitter 116, controller for light receiver 117, storage unit 119, communication unit 120, display unit 121, and emergency sound generating unit 122. Further, data processing unit 113 has an appropriate interface such as an optic fiber between itself and sensor unit 112. The connection between data processing unit 113 and sensor unit 112 is made of a material having an appropriate flexibility.

The operation of blood glucose monitoring system 110 shown in Fig. 2 will be sequentially described. Light emitter 114 is responsive to the instructions from CPU 118 of data processing unit 113 for emitting, under the control of controller of light emitter 116, light having wavelengths ranging from ultraviolet to infrared regions into finger 111 to which sensor unit 112 is attached. The light that has permeated finger 111 is received by light receiver 115 located at the other side of the finger and transferred via an optic fiber to controller of light receiver 117. Controller of light receiver 117 performs a spectrum analysis of the light and sends the data regarding the received light having a narrow range of wavelengths to CPU 118 using a light sensor and an analog to digital converter both incorporated therein.

CPU 118 analyzes the data regarding the received light from controller of light receiver 117 to obtain the components of the blood from the wavelengths absorbed and the amount of absorption while the light passes through finger 111, thereby calculating the person's blood glucose level as one of the components. The above-mentioned process of emission, receipt and analysis of light is repeated.

The blood glucose value thus obtained is compared with the person's blood glucose data in the past which is stored in storage 119. If the newly detected blood glucose value is outside the normal range or outside a predetermined range for a predetermined time interval, then an attention signal is transmitted via communication unit 120 to central unit 130 at a medical institute which functions as a center of the medical network of the area. The result of the above comparison is provided to the person him/herself visually on display 121 and audibly via emergency sound generation unit 122. On display 121, the normal blood glucose range of the person and the current value will be displayed.

The detection of the blood glucose level by the present invention's system 110 may be selectively arranged to function in a temporal, continuous or intermittent manner by setting the timing of data processing unit 113. When the blood glucose level is monitored in a continuous or intermittent manner, it is determined whether the present temporal change of the blood glucose level is medically dangerous by comparing the currently occurring change with the past data stored in the database of storage 119. When necessary, an alarm will be generated.

The mechanism of sensor unit 112 may be selected from a variety of existing systems. Namely, sensor 112 may comprise means for detecting vital signs other than blood glucose level such as pulse wave, oxygen saturation in the arterial blood (SpO2), hematocrit and body temperature. In such cases, the blood glucose level can be indirectly calculated from one or more vital signs other than the blood glucose level. The most appropriate blood glucose level may be selected from the blood glucose levels detected at different moments by comparing the values directly calculated for example as a result of the spectrum analysis above with the levels indirectly calculated in other ways. The accuracy of the blood glucose level detected at one moment may also be improved by using one or more other vital signs detected at the moment.

Sensor unit 112 may detect a plurality of vital signs with a single sensor by time wise varying the wavelength of the light emitted from the light emitter 114. Since sensor unit 112 is directly attached on the person's body, for example, on a finger, the unit has to be small sized and it is difficult to include more than one sensor therein. Thus it is preferable for the system of sensor unit 112 to be arranged to be set by different configuration of microprocessor 118. In particular, it may be possible for microprocessor 118 to be configured as an ASIC which includes all the functions of the blood glucose monitoring system in accordance with the present invention.

Sensor unit 112 of system 110, in addition to the sensor for detecting the blood glucose level, includes a sensor detecting bias data. By analyzing the differences of the bias data at the data processing unit, the accuracy of the already calculated blood glucose level may be improved. Further, sensor unit 112 comprises light emitting means for generating light having wavelengths ranging from ultraviolet to infrared regions and means for separating and detecting the light in each wavelength and data processing unit 113 is capable of calculating the blood glucose level of the person on the basis of the separated and detected data on the light intensities of a plurality of wavelength regions. Sensor unit 112 also comprises light emitting means for generating light, while continuously changing the light having wavelengths ranging from ultraviolet to infrared regions and data processing unit 113 is capable of calculating the blood glucose level of the person on the basis of the light wavelengths and intensities detected by the light emitting means while excluding the influences of the materials included in the blood.

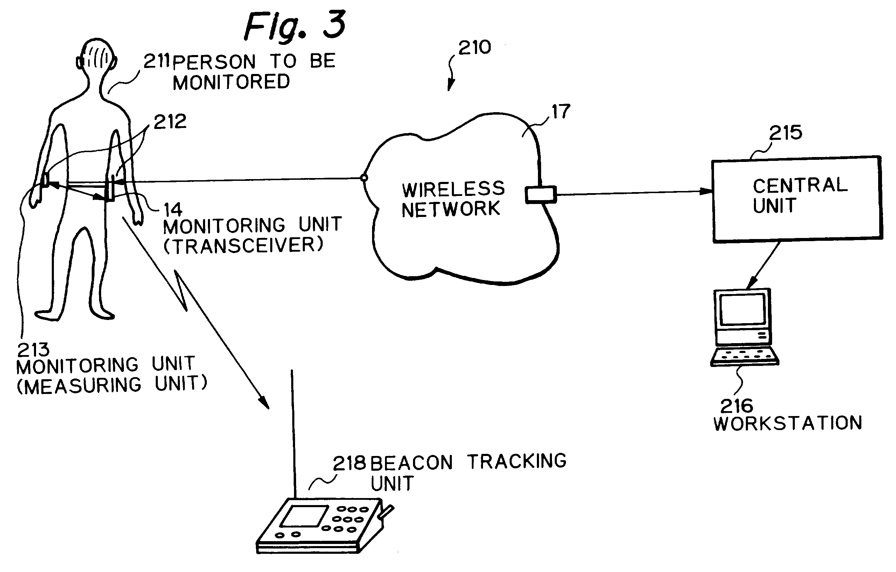

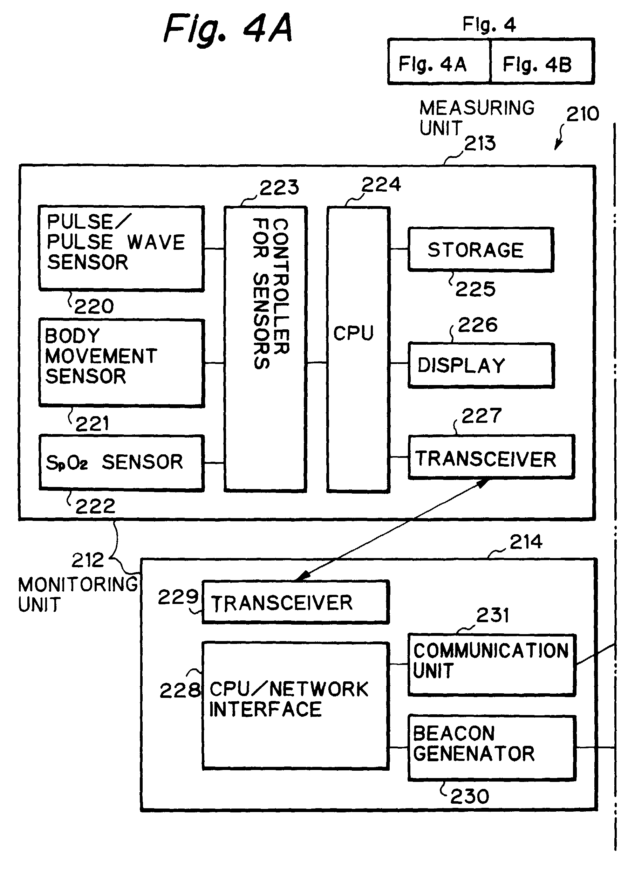

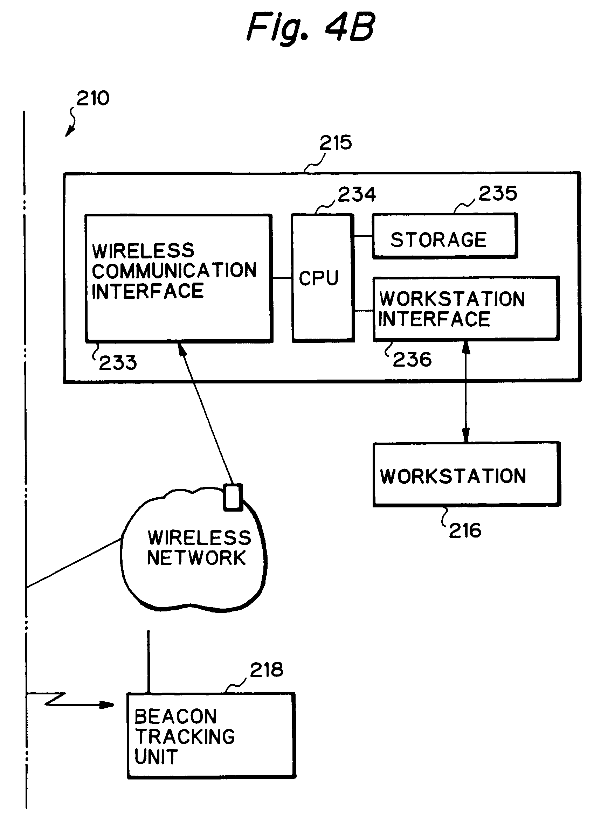

The location and physical condition monitoring system which is the second embodiment of the present invention will now be described by referring to Figs. 3 and 4. Fig. 3 shows a perspective block diagram of location and physical condition monitoring system 210. Monitoring system 210 comprises monitoring unit 212 directly attached to the body of the person being monitored, central unit 215 installed at a medical institute of the area and radio (wireless communication) network 217 which transfers data between these two units. Central unit 215 is a computer system operating for 24 hours a day, 365 days a year which communicates with a plurality of monitoring units 212 attached to people living in the area. Further, central unit 215 communicates via a radio network with computer terminals on which physicians, nurses and personnel of emergency vehicles work (for example, workstations 216 with a communication capability). When an emergency situation arises, these medical personnel will generate an appropriate instruction by using the communication function of his/her own workstation 216.

Monitoring unit 212 comprises measuring unit 213 for measuring physical data of the person and transceiver 214 for performing radio communication. Transceiver 214 functions to identify the ID of an antenna and to search the radio waves transmitted from measuring unit 212 by using beacon tracking unit 218. The operation of the system 210 may be initiated by either of central unit 215 and monitoring unit 212. System 210 further includes a recognition capability by sound.

The information regarding the location and physical condition of the person being monitored which has been detected by measuring unit 213 will be transferred to central unit 215 via radio network 217. In actually searching the location of the person, beacon tracking unit 218 is installed into an emergency vehicle (not shown) to track the radio waves transmitted from monitoring unit 212 so that the vehicle will search the source of the beacon waves while moving, thereby reaching the location of the person.

Fig. 4 shows monitoring unit 212 and central unit 215 of system 210 in more detail. The same elements of Fig. 4 as in Fig. 3 are identified by the same reference numerals. In the embodiment shown in Fig. 4, measuring unit 213 is attached to a wrist and transceiver 214 is attached to the waist of the person. These units may be attached to other part of the body as long as the attachment of these units will not restrict the ordinary activities of the person too much. However, measuring unit 213 has to be in contact with the skin since the physical data is measured directly from the skin. Measuring unit 213 comprises pulse wave/pulse rate sensor 220, body movement sensor 221, SpO2 sensor 222, controller for the sensors 223, CPU 224, storage 225, display 226, and transceiver 227 for communicating with transceiver 230 in monitoring unit 212 by means of week radio waves. The vital signs detected by sensors 220, 221, 222 will be analyzed by CPU 224 and storage 223. When an abnormality is found, it will be communicated to communication unit 214 via transceiver 227 and further to central unit 215 of the medical institute.

Measurements of the parameters will be made in accordance with the following method while considering that measuring unit 213 is small in size and attached to the person's body in such a manner that does not compromise the QOL of the person. The present invention may be characterized by the fact that this method is not used alone but is incorporated into the remote monitoring system which can monitor the physical condition and locate the person.
1) Measuring unit 213 includes a pressure change detection transducer which is connected to a gas-enclosing air-pad or a pad made of flexible material attached to the person's body and which is capable of detecting the person's pulse wave or pulse rate and this air-pad may be adapted to remove noise caused by body movement or at least lower it.
2) Measuring unit 213 comprises a very thin-film resistor or thermistor which is attached to the person's body for detecting the pulse wave and pulse rate by measuring the temperature changes rather than by detecting the pressure changes.
3) Measuring unit 213 comprises a light emitter and a light receiver by which the light having the wavelength from a visible region to an infrared region or having a plurality of wavelengths is permeated through, diffused in or reflected from a part of the person's body, thereby measuring the pulse wave, pulse rate or SpO2 from the absorption spectrum.
4) Measuring unit 213 comprises a condenser microphone for detecting sound waves generated by the blood flow, thereby measuring the pulse wave and pulse rate of the person.
5) Measuring unit 213 comprises an acceleration sensor for detecting a person's body movement.
6) Measuring unit 213 preferably comprises at least one of an amplifier whose output is in a logarithmic form and an amplifier which automatically adjust its level so that the saturation caused by the noise because of the body movement can be avoided.
7) CPU (223,224) of the measuring unit 213 preferably comprises: means for calculating the first and second derivative of the detected pulse wave data; means for separating the pulse wave by comparing it with patterns of the pulse wave forms; and means for improving the accuracy of the pulse wave forms by comparing them to the data detected by the body movement sensor.

These methods are merely illustrative and by no means limit the present invention. As far as it is possible considering the invention's structure, any other method may be employed.

CPU 224 determines whether the person is in a normal condition or not on the basis of a variety of normal ranges particularly set to this specific person which are stored in storage 223. Since monitoring unit 212 is small and light, the capacity of storage 223 is limited and thus it is not necessarily possible to store all the medical history covering a long period of time. However, storage 235 in central unit 215 may be of a large capacity, where a medical database having a medical history for a long time period of the person being monitored is constructed. From this bulk of data, a normal range of a specific physical parameter P of an individual A may be set as, for example, X≦P≦Y by a physician. This range may differ from one individual to another and it is an advantage of the invention that this range can be customized. This normal range X≦P≦Y is transmitted to and stored in storage 225 of monitoring unit that individual A carries. The range may be updated via the radio network. The value of parameter P measured by measuring unit 213 is out side of the range X≦P≦Y, CPU 224 generates an emergency alarm as discussed above.

Measuring unit 213 transmits a normal signal to transceiver at a predetermined time interval and further to central unit 215. By this normal signal, the medical institute is able to recognize that the physical condition of the person 211 is normal. Further, measuring unit 213 has the time and condition displayed on display 226. Communication unit 214 of monitoring unit 212 comprises CPU/radio network interface 228, transceiver 227 and beacon generator 230 and receives signal from measuring unit 213 at transceiver 229 and transmits the signal representative of the location and physical condition data under the control of the PHS module to central unit from transceiver.

When the communication is initiated by central unit 215, a variety of instructions will be received by transceiver 230 and location and/or physical condition data will be transmitted to central unit 215.

Central unit 215 comprises radio communication interface 233, CPU 234, storage 235 and I/O interface 236. Central unit 215 further communicates with a workstation 216 located at the individual physician's workplace via a radio network. Radio communication interface 233 inputs into CPU 234 the data and information transmitted from transceiver 214 of monitoring unit 212 via radio network 217 and computers for the network 232. CPU 234 and storage 235 function to analyze, determine and store the data and control signal included in the received information. The result of the analysis is transferred to workstation 216 via workstation interface 236 and is provided to physicians, nurses and personnel of emergency vehicles. On the contrary, the physician, nurses and personnel of the emergency vehicles provide instructions form their workstations to CPU 234 of central unit 215 and then CPU 234 collects the physical data by so instructing transceiver 214 of monitoring unit 214 via radio interface 233 and radio network 217. The monitoring system of the invention is capable of communicating with only transceiver 214 in monitoring unit 212. It is also possible to orally communicate between the person and the medical personnel at workstation 216 under the control of central unit 215.

The detection of the location of the person will be done in accordance with the following method while considering that measuring unit 213 is small in size and attached to the person's body in such a manner that does not lower the QOL of the person. The present invention is also characterized by the fact that this method is not used alone but is incorporated into the monitoring system.
1) The location detection may be done by means of the Global Positioning System (GPS).
2) The location detection may be done by means of a plurality of satellites of a satellite communication system (such as the Iridium Project).
3) The information regarding location and physical condition of the person is obtained by using the PHS network, a satellite network, cellular telephone network , bi-directional pager network or private network and by initiating the system form either monitoring unit 212 or central unit 215.

Incidentally, measuring unit 213 of monitoring unit 212 and transceiver 214 are in different housing. Measuring unit 213 is attached to a wrist and transceiver 214 to the waist of the person for example. Communication between these two units is effected done by means of a low level radio wave or electro-magnetic induction. These two units may be incorporated into a single housing and attached to a part (for example, a wrist)of the body.

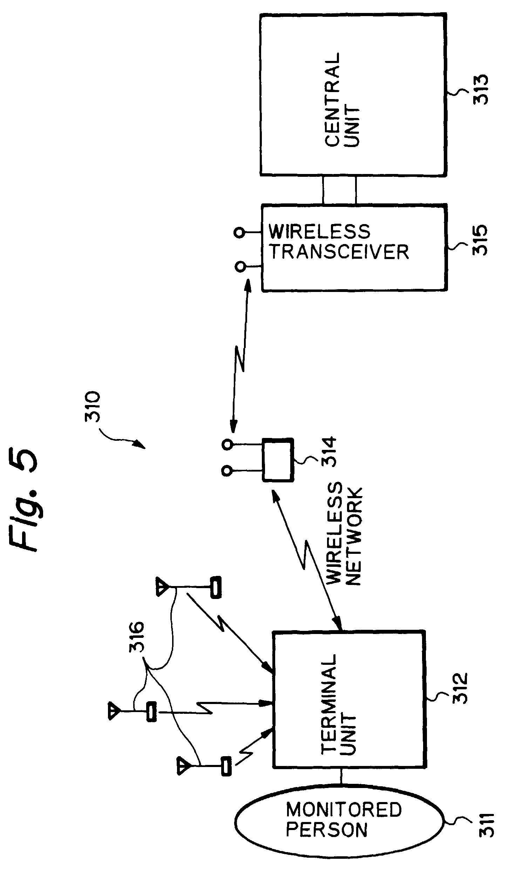

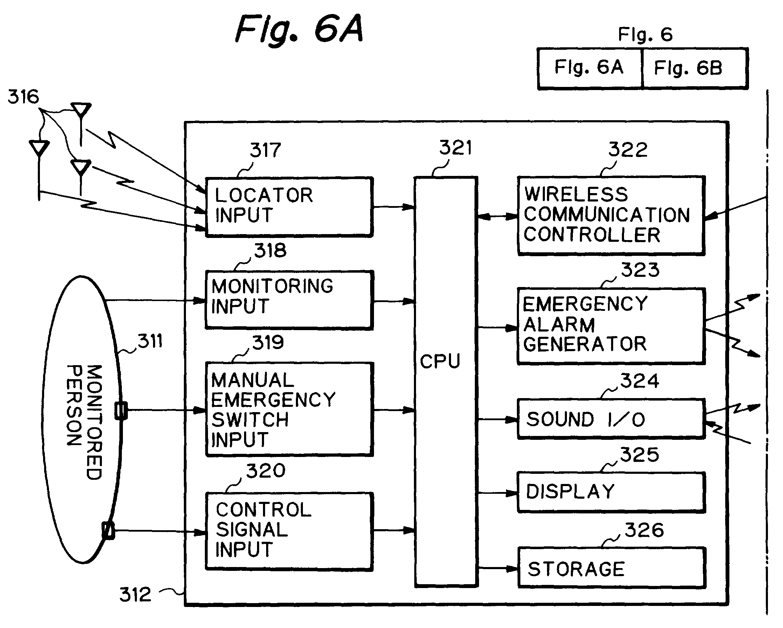

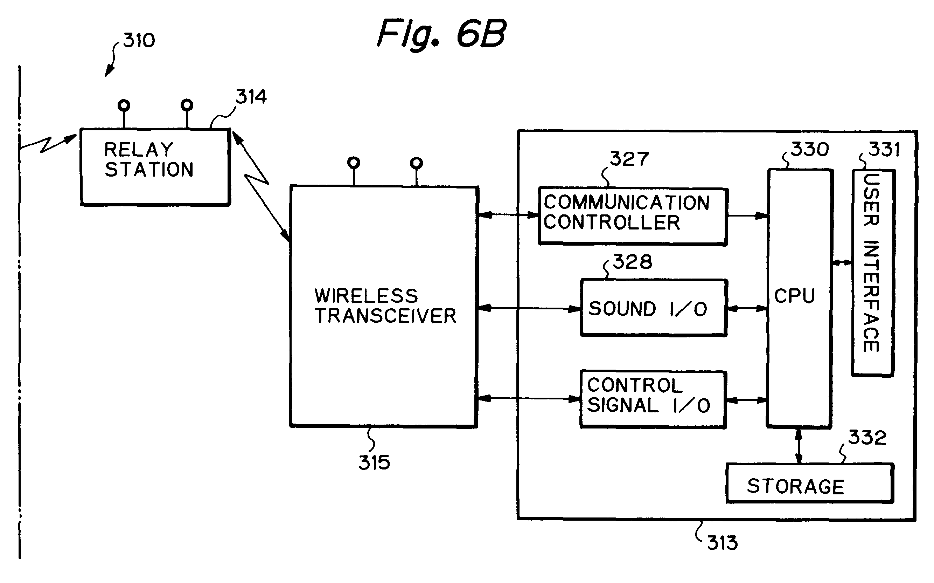

The third embodiment of the present invention will now be described by referring to Figs. 5 and 6. Though this embodiment resembles the second embodiment, the third embodiment is realized as a monitoring system for remotely monitoring and taking care of a person staying at home.

Fig. 5 is a perspective diagram of system 310 for remotely taking care of a person at home which is the third embodiment of the present invention. System 310 comprises terminal unit 312 directly attached to, for example, to a wrist of person 311 being taken care of, central unit 313 located at a medical institute which is in charge of medical care of the area and a radio network capable of bi-directional communication between these two units.

Radio network comprises radio (wireless) transceiver 315 including a transmitter and a receiver on the side of central unit 313 and one or more relay stations are set up depending upon the geometrical situation of the area in order to smoothly realize radio communication among all the people being remotely taken care of in the area. A communication satellite (not shown) may be used instead. Terminal unit 312 at the house of the person includes radio communication controller 322 which comprises a transmitter and a receiver.

Further, terminal unit 312 receives radio waves from a plurality of radio waves transmitters 316 so that the current location of person 311 is detected on the basis of the triangulation principle.

In Fig. 6, terminal unit 312 and central unit 313 are illustrated in more detail. The same reference numerals are attached to the same elements as in Fig. 5. Terminal unit 312 is directly attached to a wrist of person 311. Terminal unit 312 continuously or intermittently monitors the physical condition of the person 311 by detecting physical data from the person's body by means of monitoring input 318.

All the detection of the data is effected in the un-invasive manner. For example, the pulse wave may be detected on the basis of a detected body temperature or by using light such as an infrared light. In such cases, a sensor for detecting pulse wave may be used. In order to detect the pulse wave while keeping the power consumption to the minimum level, a very thin-film thermocouple is used in this embodiment. Further, SpO2 can be un-invasively detected by using a light such as an infrared light and visible light. Electrocardiogram can also be detected.

Since the processing operation of the physical data by CPU 321 in this third embodiment is similar to those in the monitoring systems of the first and second embodiments, the same description will not be repeated here. However, in addition to the already described features, terminal unit 312 is able to detect whether the unit is attached to the body of the person, so that the medical personnel at the medical institute will know that the unit is currently not worn by the person.

While three illustrative embodiments of the invention have been shown and described, numerous variations and alternate embodiment will occur to those skilled in the art. Such variations and alternate embodiments are contemplated, and can be made without departing from the spirit and scope of the invention as defined in the appended claims.

According to its broadest aspect the invention relates to a blood glucose monitoring system comprising: sensor means attached on a part of a person for detecting data representative of the blood glucose level of the person, the sensor means having a light emitter module and a light receiver module; data processor means for calculating the blood glucose level of the person on the basis of the light data transferred from said sensor means, the data processor means having a microprocessor incorporated for controlling said sensor means and analyzing the data received by the light receiver; and storage means for storing a database consisting of the physical data of the person, the physical data including the blood glucose level of the person for a certain time period in the past.

It should be noted that the objects and advantages of the invention may be attained by means of any compatible combination(s) particularly pointed out in the items of the following summary of the invention and the appended claims.

### SUMMARY OF THE INVENTION

1. A blood glucose monitoring system comprising:
   sensor means attached on a part of a person for detecting data representative of the blood glucose level of the person, the sensor means having a light emitter module and a light receiver module;
   data processor means for calculating the blood glucose level of the person on the basis of the light data transferred from said sensor means, the data processor means having a microprocessor incorporated for controlling said sensor means and analyzing the data received by the light receiver; and
   storage means for storing a database consisting of the physical data of the person, the physical data including the blood glucose level of the person for a certain time period in the past;
   said data processor means comprising means for comparing the calculated blood glucose level with data in the database, means for accessing, on the basis of the data comparison, possible effects that the calculated blood glucose level may have on the person's body, and means for instructing the monitoring system to generate an alarm signal when the effects exceed a predetermined threshold level that should be judged as being medically dangerous.
2. The blood glucose monitoring system
   wherein both said sensor means and said data processor means can be attached on a part of the person's body including a finger and a wrist.
3. The blood glucose monitoring system
   wherein said microprocessor controls the timing of the blood glucose detection by said sensor means so that said sensor means detects the blood glucose level in a temporary, continuous or intermittent manner.
4. The blood glucose monitoring system further comprising:
   means for determining, when said sensor means performs the blood glucose monitoring in a continuous or intermittent manner, whether the changes in the blood glucose level during a certain time period should be judged as being abnormal on the basis of the past changes in the blood glucose level stored in the database; and
   means for generating an alarm signal when the changes in the blood glucose level exceed a predetermined threshold level that should be judged as being medically dangerous.
5. The blood monitoring system
   wherein said data processor means further comprises means for selecting the blood glucose level most appropriate in the arterial blood flow from among a plurality of blood glucose levels detected at a plurality of moments.
6. The blood monitoring system
   wherein said sensor means further comprises means for detecting one or more vital signs of the pulse wave, the oxygen saturation level in the arterial blood (SpO2), the hematocrit (Hct) and the vital signs including fluctuation of the body temperature besides the blood glucose level and said data processor means comprises means for calculating the blood glucose level with the detected one or more vital signs and means for improving the accuracy of the calculated blood glucose level.
7. The blood monitoring system
   wherein said sensor means further comprises means for time wise changing the wavelength of the light emitted by said means for detecting one or more vital signs in said sensor means, so that said sensor means is capable of detecting more than one vital sign by using a single sensor.
8. The blood monitoring system
   wherein said sensor means further comprising a sensor for detecting bias data in addition to the sensor for detecting the blood glucose level and said data processor means comprises means for improving the accuracy of the already calculated blood glucose level by analyzing the differences between the detected data and the bias data.
9. The blood monitoring system
   wherein said data processing means comprises means for generating a visual or audio alarm on the basis of the alarm signal and means for transmitting an alarm to a medical institute via a network having an interface with said data processing means.
10. The blood monitoring system
   wherein said sensor means comprises light emitting means for generating light having wavelengths ranging from ultraviolet to infrared regions and means for separating and detecting the light in each wavelength and said data processing means further comprises means for calculating the blood glucose level of the person on the basis of the separated and detected data on the light intensities of a plurality of wavelength regions.
11. The blood monitoring system
   wherein said sensor means comprises light emitting means for generating light while continuously changing the light having wavelengths ranging from ultraviolet to infrared regions and said data processing means further comprising means for calculating the blood glucose level of the person on the basis of the light wavelengths and intensities detected by the light receiving means while excluding the influences of the materials included in the blood and tissue.
12. The blood monitoring system
   wherein said light emitter comprises light emitting means for generating light having wavelengths ranging from ultraviolet to infrared regions and said data processing means further comprises means for performing a spectrum analysis of the light which has permeated a part of the person's body and has been detected by the light receiver and for calculating the light absorption at each wavelength.
13. The blood monitoring system
   wherein said data processing means further comprises means for improving the accuracy of the calculated blood glucose level by analyzing the changes in the currently detected data on the basis of the comparison to the past data relating the components in the blood of the person stored in the database.
14. A computer-readable storage medium where a computer software is stored which performs the functions of the blood glucose monitoring system recited by all the preceding claims.
15. A system for remotely monitoring the present location and physical condition of a person on real time basis, the system comprising:
   a central unit which is a computer system for performing the remote monitoring on real time basis;
   monitoring means attached on the body of the person for detecting parameters representative of the physical condition of the person; and
   wireless communication means located in the neighborhood of said monitoring means or integrated with said monitoring means for communicating with said central unit;
      wherein the monitoring unit consisted of said monitoring means and said wireless communication means is responsive to the actuation of an emergency switch by the person for transmitting or automatically transmits, in a wireless manner, a signal representative of the present location and physical condition of the person to said central unit and the monitoring unit is also responsive to the instructions from said central unit for transmitting a signal representative of the present location and physical condition of the person to said central unit and
      wherein said monitoring system comprises audio communication means for exchanging audio communications with said central unit.
16. The system wherein said monitoring unit uses the PHS communication network as a location search means to communicate with the central unit, the system further comprising:
   means for detecting and transmitting to the central unit the ID of the PHS antenna which is currently communicating and for having the central unit specify the location of the antenna from the detected ID of the antenna;
   transmitter means for transmitting a radio wave having a particular wavelength; and
   means for searching the source of the radio wave other than said monitoring unit.
17. The system wherein the present location of the person is detected by means of the Global Positioning System (GPS).
18. The system wherein the present location of the person is detected by means of a satellite communication system including the Iridium Project.
19. The system wherein said wireless communication system network includes the PHS communication network, a cellular telephone network, a satellite communication network, bi-directional pager network and private wireless networks and wherein the information regarding at least one of the present location and physical condition of the person is transmitted form said monitoring unit to said central unit by being initiated by either of the monitoring unit or the central unit.
20. The system wherein said monitoring unit is a pressure fluctuation detection transducer which is connected to a gas-enclosing pad or a pad made of flexible material attached to the person's body and which is capable of detecting the person's pulse wave or pulse rate and wherein said wireless communication means comprises means for transmitting a signal representative of the location to the central unit when at least one of the detected pulse wave and pulse rate is outside of a predetermined normal range.
21. The system wherein said monitoring unit comprises a very thin-film resistor or thermistor which is attached to the person's body for detecting the pulse wave and pulse rate by measuring the temperature changes.
22. The system wherein said monitoring unit comprises a light emitter module and a light receiver module by which the light having the wavelength from a visible region to an infrared region or having a plurality of wavelengths is permeated through, diffused in or reflected from a part of the person's body, thereby measuring the pulse wave, pulse rate or SpO2 by the absorption spectrum and monitoring the physical condition of the person's body.
23. The system wherein said monitoring unit comprises a condenser microphone for detecting the sound wave generated by the blood flow, thereby measuring the pulse wave and pulse rate of the person.
24. The system wherein said monitoring unit comprises an acceleration sensor for detecting the person's body movement.
25. The system wherein said gas-enclosing pad or a pad made of flexible material comprises means for decreasing a low-frequency noise caused by the body movement.
26. The system wherein said monitoring unit comprises at least one of an amplifier whose output is in a logarithmic form and an amplifier which automatically adjust its level so that the saturation caused by the noise because of the body movement can be avoided.
27. The system wherein the controller of said monitoring unit comprises:
   means for calculating the first and second derivative of the detected pulse wave data;
   means for separating the pulse wave by comparing it with patterns of the pulse wave forms; and
   means for improving the accuracy of the pulse wave forms by comparing them to the data detected by the body movement sensor.
28. The system wherein said monitoring unit and said communication unit are in a different housing and each of these unit has means for communicating with each other by means of a low level radio wave or electro-magnetic induction.
29. The system wherein each of said central unit and said monitoring unit has means for assuring at a predetermined time interval that these units are in a normal condition.
30. The system wherein said monitoring unit transmits a normal condition assuring signal and data regarding pulse wave and SpO2 to said central unit, so that the central unit stores and analyzes the data.
31. The system wherein said monitoring unit, in transmitting abnormality alarm signal to said central unit, transmits to said central unit at least one of the pulse wave and pulse rate as a vital sign or a vital sign just after transmitting an abnormality signal.
32. The system wherein said transmitter means comprises means for transmit an radio wave including a code identifying the person being monitored and wherein said search means comprised means for identifying the person being monitored by recognizing the identification code.
33. The system wherein said detection means comprises means for detecting the electrocardiogram.
34. The system wherein the location of the person is specified by one more of the following means:
   means for detecting the location of the person on the basis of the PHS communication network and location information of the PHS antenna currently communicating with the person;
   means based on a beacon system for searching the radio wave generated by the person;
   means for using the Global Positioning System; and means for using private radio waves generated by a plurality of communication stations.
35. The system wherein an emergency alarm signal can be generated manually by person's actuating a switch on the unit.
36. The system wherein, when it is determined that the person is in an emergent condition, in addition to the generation of a radio-wave emergent signal, a sound is continuously generated so that such a condition can be recognized by people nearby.
37. The system wherein said monitoring unit has means for assuring that these units are in a normal condition by transmitting a signal representative of a normal condition at a predetermined time interval when the detected physical condition data is in a normal range.
38. The system further comprising means for, when said emergency alarm signal is transmitted or the system is driven by the central unit, transmitting the physical condition data and/or present location data of the person being monitored for the predetermined time period in the past and/or for the time period thereafter from the monitoring unit to the central unit.
39. The system wherein, when the location of the person is determined on the basis of the private radio wave from the plurality of communication stations, the radio wave is received which is synchronously transmitted from the stations and the time required for the radio wave to reach the location is used to calculate the distances form the stations on the principle of triangulation.

## Claims

1. A blood glucose monitoring system comprising:
sensor means attached on a part of a person for detecting data representative of the blood glucose level of the person, the sensor means having a light emitter module and a light receiver module;
data processor means for calculating the blood glucose level of the person on the basis of the light data transferred from said sensor means, the data processor means having a microprocessor incorporated for controlling said sensor means and analyzing the data received by the light receiver; and
storage means for storing a database consisting of the physical data of the person, the physical data including the blood glucose level of the person for a certain time period in the past;
said data processor means comprising means for comparing the calculated blood glucose level with data in the database, means for accessing, on the basis of the data comparison, possible effects that the calculated blood glucose level may have on the person's body, and means for instructing the monitoring system to generate an alarm signal when the effects exceed a predetermined threshold level that should be judged as being medically dangerous.

2. The blood glucose monitoring system recited by claim 1 wherein both said sensor means and said data processor means can be attached on a part of the person's body including a finger and a wrist,
and/or wherein preferably 1 wherein said microprocessor controls the timing of the blood glucose detection by said sensor means so that said sensor means detects the blood glucose level in a temporary, continuous or intermittent manner,
and/or further preferably
comprising:
means for determining, when said sensor means performs the blood glucose monitoring in a continuous or intermittent manner, whether the changes in the blood glucose level during a certain time period should be judged as being abnormal on the basis of the past changes in the blood glucose level stored in the database; and
means for generating an alarm signal when the changes in the blood glucose level exceed a predetermined threshold level that should be judged as being medically dangerous,
and/or wherein preferably
said data processor means further comprises means for selecting the blood glucose level most appropriate in the arterial blood flow from among a plurality of blood glucose levels detected at a plurality of moments,
and/or wherein preferably
said sensor means further comprises means for detecting one or more vital signs of the pulse wave, the oxygen saturation level in the arterial blood (SpO2), the hematocrit (Hct) and the vital signs including fluctuation of the body temperature besides the blood glucose level and said data processor means comprises means for calculating the blood glucose level with the detected one or more vital signs and means for improving the accuracy of the calculated blood glucose level,
and/or wherein preferably wherein said sensor means further comprises means for time wise changing the wavelength of the light emitted by said means for detecting one or more vital signs in said sensor means, so that said sensor means is capable of detecting more than one vital sign by using a single sensor.

3. The blood monitoring system recited by claim 1 wherein said sensor means further comprising a sensor for detecting bias data in addition to the sensor for detecting the blood glucose level and said data processor means comprises means for improving the accuracy of the already calculated blood glucose level by analyzing the differences between the detected data and the bias data,
and/or wherein preferably
said data processing means comprises means for generating a visual or audio alarm on the basis of the alarm signal and means for transmitting an alarm to a medical institute via a network having an interface with said data processing means,
and/or wherein preferably
said sensor means comprises light emitting means for generating light having wavelengths ranging from ultraviolet to infrared regions and means for separating and detecting the light in each wavelength and said data processing means further comprises means for calculating the blood glucose level of the person on the basis of the separated and detected data on the light intensities of a plurality of wavelength regions,
and/or wherein preferably
said sensor means comprises light emitting means for generating light while continuously changing the light having wavelengths ranging from ultraviolet to infrared regions and said data processing means further comprising means for calculating the blood glucose level of the person on the basis of the light wavelengths and intensities detected by the light receiving means while excluding the influences of the materials included in the blood and tissue,
and/or wherein preferably
said light emitter comprises light emitting means for generating light having wavelengths ranging from ultraviolet to infrared regions and said data processing means further comprises means for performing a spectrum analysis of the light which has permeated a part of the person's body and has been detected by the light receiver and for calculating the light absorption at each wavelength,
and/or wherein preferably
said data processing means further comprises means for improving the accuracy of the calculated blood glucose level by analyzing the changes in the currently detected data on the basis of the comparison to the past data relating the components in the blood of the person stored in the database.

4. A computer-readable storage medium where a computer software is stored which performs the functions of the blood glucose monitoring system recited by all the preceding claims.

5. A system for remotely monitoring the present location and physical condition of a person on real time basis, the system comprising:
a central unit which is a computer system for performing the remote monitoring on real time basis;
monitoring means attached on the body of the person for detecting parameters representative of the physical condition of the person; and
wireless communication means located in the neighborhood of said monitoring means or integrated with said monitoring means for communicating with said central unit;
wherein the monitoring unit consisted of said monitoring means and said wireless communication means is responsive to the actuation of an emergency switch by the person for transmitting or automatically transmits, in a wireless manner, a signal representative of the present location and physical condition of the person to said central unit and the monitoring unit is also responsive to the instructions from said central unit for transmitting a signal representative of the present location and physical condition of the person to said central unit and
wherein said monitoring system comprises audio communication means for exchanging audio communications with said central unit.

6. The system recited by claim 15 wherein said monitoring unit uses the PHS communication network as a location search means to communicate with the central unit, the system further comprising:
means for detecting and transmitting to the central unit the ID of the PHS antenna which is currently communicating and for having the central unit specify the location of the antenna from the detected ID of the antenna;
transmitter means for transmitting a radio wave having a particular wavelength; and
means for searching the source of the radio wave other than said monitoring unit,
and/or wherein preferably the present location of the person is detected by means of the Global Positioning System (GPS),
and/or wherein preferably the present location of the person is detected by means of a satellite communication system including the Iridium Project,
and/or wherein preferably said wireless communication system network includes the PHS communication network, a cellular telephone network, a satellite communication network, bi-directional pager network and private wireless networks and wherein the information regarding at least one of the present location and physical condition of the person is transmitted form said monitoring unit to said central unit by being initiated by either of the monitoring unit or the central unit,
and/or wherein preferably said monitoring unit is a pressure fluctuation detection transducer which is connected to a gas-enclosing pad or a pad made of flexible material attached to the person's body and which is capable of detecting the person's pulse wave or pulse rate and wherein said wireless communication means comprises means for transmitting a signal representative of the location to the central unit when at least one of the detected pulse wave and pulse rate is outside of a predetermined normal range,
and/or wherein preferably said monitoring unit comprises a very thin-film resistor or thermistor which is attached to the person's body for detecting the pulse wave and pulse rate by measuring the temperature changes,
and/or wherein preferably said monitoring unit comprises a light emitter module and a light receiver module by which the light having the wavelength from a visible region to an infrared region or having a plurality of wavelengths is permeated through, diffused in or reflected from a part of the person's body, thereby measuring the pulse wave, pulse rate or SpO2 by the absorption spectrum and monitoring the physical condition of the person's body,
and/or wherein preferably said monitoring unit comprises a condenser microphone for detecting the sound wave generated by the blood flow, thereby measuring the pulse wave and pulse rate of the person.

7. The system recited by claim 5 wherein said monitoring unit comprises an acceleration sensor for detecting the person's body movement,
and/or wherein preferably said gas-enclosing pad or a pad made of flexible material comprises means for decreasing a low-frequency noise caused by the body movement,
and/or wherein preferably said monitoring unit comprises at least one of an amplifier whose output is in a logarithmic form and an amplifier which automatically adjust its level so that the saturation caused by the noise because of the body movement can be avoided,
and/or wherein preferably the controller of said monitoring unit comprises:
means for calculating the first and second derivative of the detected pulse wave data;
means for separating the pulse wave by comparing it with patterns of the pulse wave forms; and
means for improving the accuracy of the pulse wave forms by comparing them to the data detected by the body movement sensor,
and/or wherein preferably said monitoring unit and said communication unit are in a different housing and each of these unit has means for communicating with each other by means of a low level radio wave or electro-magnetic induction,
and/or wherein preferably each at said central unit and said monitoring unit has means for assuring at a predetermined time interval that these units are in a normal condition.

8. The system recited by claim 5 wherein said monitoring unit transmits a normal condition assuring signal and data regarding pulse wave and SpO2 to said central unit, so that the central unit stores and analyzes the data,
and/or wherein preferably said monitoring unit, in transmitting abnormality alarm signal to said central unit, transmits to said central unit at least one of the pulse wave and pulse rate as a vital sign or a vital sign just after transmitting an abnormality signal,
and/or wherein preferably said transmitter means comprises means for transmit an radio wave including a code identifying the person being monitored and wherein said search means comprised means for identifying the person being monitored by recognizing the identification code,
and/or wherein preferably said detection means comprises means for detecting the electrocardiogram.

9. The system recited by claim 5 wherein the location of the person is specified by one more of the following means:
means for detecting the location of the person on the basis of the PHS communication network and location information of the PHS antenna currently communicating with the person;
means based on a beacon system for searching the radio wave generated by the person;
means for using the Global Positioning System; and
means for using private radio waves generated by a plurality of communication stations,
and/or wherein preferably an emergency alarm signal can be generated manually by person's actuating a switch on the unit,
and/or wherein preferably when it is determined that the person is in an emergent condition, in addition to the generation of a radio-wave emergent signal, a sound is continuously generated so that such a condition can be recognized by people nearby,
and/or wherein preferably said monitoring unit has means for assuring that these units are in a normal condition by transmitting a signal representative of a normal condition at a predetermined time interval when the detected physical condition data is in a normal range,
and/or further preferably comprising means for, when said emergency alarm signal is transmitted or the system is driven by the central unit, transmitting the physical condition data and/or present location data of the person being monitored for the predetermined time period in the past and/or for the time period thereafter from the monitoring unit to the central unit,
and/or wherein preferably when the location of the person is determined on the basis of the private radio wave from the plurality of communication stations, the radio wave is received which is synchronously transmitted from the stations and the time required for the radio wave to reach the location is used to calculate the distances form the stations on the principle of triangulation.

10. A blood glucose monitoring system comprising:
sensor means attached on a part of a person for detecting data representative of the blood glucose level of the person, the sensor means having a light emitter module and a light receiver module;
data processor means for calculating the blood glucose level of the person on the basis of the light data transferred from said sensor means, the data processor means having a microprocessor incorporated for controlling said sensor means and analyzing the data received by the light receiver; and
storage means for storing a database consisting of the physical data of the person, the physical data including the blood glucose level of the person for a certain time period in the past .
